# EUROPEAN PATENT APPLICATION

(11) **EP 3 108 904 A1**
(43) Date of publication of application: **28.12.2016**
(21) Application number: 15751603.0
(22) Date of filing: 18.02.2015
(51) Int. Cl.: A61L 27/58, A61F 2/28, B05D 1/00

(54) **DISCONTINUOUS COATING METHOD USING A BIOABSORBABLE AND BIOACTIVE BIOMATERIAL APPLIED TO SOLID SUBSTRATES, DISCONTINUOUS COATING AND USE THEREOF**

(30) Priority: 19.02.2014 BR 1403817
(71) Applicant: Fundação Universidade Federal De São Carlos, 13565-905 São Carlos - SP (BR)
(72) Inventor: ZANOTTO, Edgar Dutra, 13565-905 São Carlos - SP (BR); FILHO, Oscar Peitl, 13565-905 São Carlos - SP (BR); CHINAGLIA, Clever Ricardo, 13565-905 São Carlos - SP (BR)
(74) Representative: Zinkler, Franz
(86) International application number: PCT/BR2015/000029
(87) International publication number: WO 2015/123749

(57) **Abstract**

The invention relates to the development of a discontinuous coating using a bioabsorbable and bioactive biomaterial (which can be a bioceramic, a biopolymer or a bioactive composite) applied to solid (smooth, rough or porous) substrates. This discontinuous coating should be totally consumed in up to 10 days after implantation, so that in the end only the interface of the newly formed tissue with the implant remains. The proposed discontinuous coating can be obtained by any method that allows depositing a continuous layer, once the process parameters are duly adjusted. The preferred method for applying this discontinuous layer is airbrushing or air atomizing. The preferred biomaterial is bioglass.

## Description

### FIELD OF THE INVENTION

This invention applies to the Chemistry, Pharmacy, Medicine, and Material Engineering field of activity and, more specifically, to the area of implants, since it refers to the development of a discontinuous coating composed of a biomaterial (which can be a bioceramic, a biopolymer or a bioactive composite), bioabsorbable and bioactive, applied to solid (smooth, rough or porous) substrates, so that after the implant there is interface between the newly formed tissue and the surface of the solid substrate (implant) in the end of the tissue recovery process and/or local initial stimulation for development of said tissue.

This coating may be applied to dental, hip, and knee implants, bone reconstruction plates, intervertebral spacers, pedicular screws of spinal implant systems, repair of anophthalmic socket, and any implantable solid component.

### BACKGROUND OF THE INVENTION

### MOTIVATION

Since the average useful life of a full hip prosthesis hardly exceeds 15-20 years, and the number of people with implants increased and their average useful life decreased, the number of surgical procedures for implant replacement, or so-called revision surgeries, is increasingly higher. Because these surgeries are traumatic and risky to patients, it would be desirable for the average useful life of implants, both dental and orthopedic, to be approximately 40 years.

In order to address this need, a structural implant should have as its main feature not only the similarity with bone tissue of its elastic module, but also the ability to effectively interface with bone tissue, in other words, to be bioactive. Currently, there is no material that has all these characteristics, and the most used alternative is the "biofunctioning" of the surfaces of metallic implants, changing them physically and/or chemically, in order to grant the typical functional properties of biological systems.

### BACKGROUND

One of the options is to coat metallic implants with layers of bioactive ceramics. Nevertheless, these layers - specifically regarding interface, still an issue not completely solved, due to incompatibility of thermal expansion coefficients and elasticity modules of these two materials. This incompatibility is the main reason for flaws in coated implants and subsequent decrease of average useful life.

In this context, this invention proposes the coating of solid (smooth, rough or porous) substrates with "islands" of a bioactive and bioabsorbable biomaterial, preferably bioglass, so that they are consumed within a few days after implantation. In case of using bioglass, for example, the silica dissolution rate is directly related to bone growth rates. Thus, the process of full dissolution of this bioglass, as well as accelerating bone integration, would also eliminate eventual interface issues, which would lead to (1) an increase in the average useful life of said coated implants, (2) subsequent reduction of the number of revision surgeries due to the decrease in the number of flaws and, lastly, (3) increase in the quality of life of the patients with implants.

### STATE OF THE ART

The patent application PI 0200698-7 relates to a process for obtaining a continuous bioactive calcium phosphate coating on solid substrates through chemical treatment of said coatings. The coating mentioned in the referred application is continuous.

The patent application PI 0306528-6 describes a coating process through biomimetic growth with replacement of the use of bioactive glass for a sodium silicate solution during the phosphate nucleation step on the surface of the substrate, with which a bioactive coating layer is produced, which coats polymeric, metallic and ceramic materials. There is no mention regarding the characteristics of the layer (continuous or discontinuous) but the biomaterial deposited is the hydroxyapatite, which is not bioabsorbable in the same way as bioglasses, as it is approximately 4 times less bioactive than a bioglass. This means that, even if the coating is discontinuous, it is not expected that hydroxyapatite is dissolved or eliminated from the surface before the end of the tissue scarring process and, thus, the metal-ceramic interface is present and shall potentially present flaws. On the other hand, in the present invention the metal-ceramic interface is consumed by the organism in the initial scarring stages, but not before stimulating tissue formation.

The patent application US 5578086 describes a prosthesis that uses biocompatible composite material. The prosthesis may be crafted in two distinct ways: (1) dispersion of powdered hydroxyapatite throughout the surface of a continuous silicone or polyurethane rubber layer applied over the surface of the implant. After curing of the elastomeric material coating, it shall remain adhered to the implant, and the hydroxyapatite particles adhered to the coating (2). Powdered biomaterial, preferably hydroxyapatite, is mixed to the silicone or polyurethane. This mix (composite material) is applied to the surface of the implant that, after curing, shall remain adhered to it. The patent also includes bioglass as an alternative material. The adhesive elastomeric material coating, additionally to being continuous, is not bioabsorbable and, therefore, the implant shall not be in direct contact with the tissue to be formed.

The patent application US 5990380 describes percutaneous implants and bioactive coatings, including a substrate and bioactive glass fibers and/or particles distributed over the substrate at a pre-determined distance between fibers of at least 20 um. Although the distribution of bioglass is discontinuous, the coating over the implant is not, as the adhesion of bioglass to the implant is done by the use of silicone as an adhesive, in other words, there is a continuous and non-bioabsorbable silicone coating over the implant which will not be eliminated and the newly formed tissue shall not interface with the implant.

Patent PI0605628-8 relates to a product and acquisition process of a low-cost and mechanically resistant bioactive porous ceramic matrix, applicable to bone implants and grafts, in the following areas: Oral and maxillofacial, orthopedics and dentistry, composed of alumina (A12O3), hydroxyapatite ([Ca10(PO4)6(OH)2] and bioglass (SiO2, Na2O, CaO, CaO/P2O5). This patent relates to the production of a porous alumina composite with addition of bioglass and hydroxyapatite infiltrated in the alumina pores. This product is applied to bone grafts and is not related to the present invention except for use of bioglass as a bioactive material.

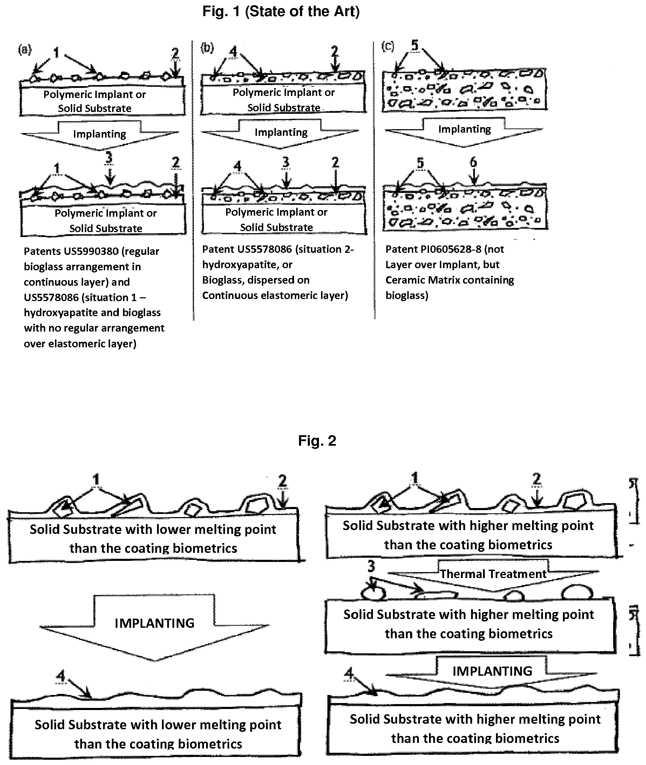

No proposals were found for coating of solid substrates using discontinuous bioactive and bioabsorbable coatings in the form presented and, for this reason, the present invention features an innovative proposal. Note that in the proposals presented in this section, and which describe layers, interface between the implant and the newly formed tissue does not take place in any of the situations and at no point in the process after implantation, in other words, in all cases implants are coated with a continuous and non-bioabsorbable layer, which intends only to fix the biomaterial particles. It should also be noted that, despite the presence of biomaterial that stimulates tissue formation, permanent layers shall remain in place, whose interfaces may potentially lead to flaws in the implant. Figures 1 and 2 show the difference between these layers and the present invention.

### OBJECT AND ADVANTAGES OF THE INVENTION

The objective of the present invention is to grant typical bioactivity of biomaterials, such as bioglasses, to any non-bioactive solid substrate without the disadvantages of continuous coatings.

The deposition of the proposed discontinuous coating solves two critical problems: (1) metal surfaces such as titanium, for example, become bioactive, and (2) eliminates the inherent possibility of flaws of continuous bioceramic coatings over metallic implants due to the fact of being a layer to be fully consumed in the initial scarring stages. This ensures the newly formed tissue directly interfaces the implant and stimulates its formation in the initial scarring stages.

In addition to eliminate any eventual issue with the interface, bioglass dissolution process also stimulates the formation of the tissue, since bioglasses feature bone producing, bone inducing and angiogenic properties which accelerate bone integration, as well as anti-inflammatory and bacteriostatic characteristics which may reduce the occurrence of post-operative infections. All these properties of bioglass shall be present on the surface of the implant while the bioglass is not fully dissolved.

### BRIEF DESCRIPTION OF THE INVENTION

The invention relates to the development of a discontinuous coating of a biomaterial (which can be a bioceramic, a biopolymer or a bioactive composite), bioabsorbable and bioactive, applied on solid (smooth, rough or porous) substrates, in order to have interface of the newly formed tissue with the implant and local stimulation for growth of said tissue in the initial scarring stages. The coating has islands of biomaterial with equivalent diameter ranges between 1 and 1200µm. For substrates with higher melting point than that of the biomaterial, particles shall adhere directly to the solid substrate due to the softening effect during the temperature increase in higher levels than the vitreous transition of the biomaterial. With substrates with lower melting point than the biomaterial particles, said particles shall adhere to its surface through use of a bioabsorbable polymer-based gel coating, preferably cellulose or collagen-based. In both cases, said coating shall be fully consumed in up to 10 days after implantation, in order to stimulate interface between the newly formed tissue and the implant, and acceleration of its initial formation.

The proposed discontinuous coating may be obtained through any method, which allows deposition of a continuous layer, once process parameters are duly adjusted, such as thermal spray, airbrushing or air atomizing, dip coating, drip coating and brush coating. The preferred method for applying this discontinuous layer is air atomizing. The reasons for this choice were: (1) low operational costs; (2) low complexity for use; (3) low residue generation; (4) minimal use of materials, which are not part of the production chain of dental or orthopedic implants, (5) safety, regarding use of non-hazardous materials or processes only, and (6) accuracy and reproducibility regarding the amount of deposited material. The thermal spray technique is an alternative for fixing biomaterial particles with lower melting point than the solid substrate. This technique generates cast biomaterial particles over the substrate, where they remain adhered.

The layer material can be any bioactive and bioabsorbable biomaterial, preferably bioglasses or bioabsorbable composites containing bioglasses, island-shaped, with size distribution in its final use form between 1 and 1200-µm and partially coating fractions of the surface of the solid substrate ranging from 2 to 80%.

The layer may be applied over any smooth, rough or porous solid substrate where, after the end of the initial tissue regeneration process, interface between the newly formed tissue and the substrate surface without a continuous layer of another material between these parts. Examples of use: dental, hip and knee implants, bone reconstruction plates, intervertebral spacers, pedicular screws of spinal implant systems, repair of anophthalmic sockets and any implantable solid substrate.

### BRIEF DESCRIPTION OF THE DRAWINGS

Figure 1 shows a schematic illustration of the layers described in the state of the art. 1 - Biomaterial particles - Bioglass or hydroxyapatite, over the continuous layer of adhesive material; 2 - Continuous adhesive layer which will join the implant to the biomaterial; 3 - Layer of newly formed tissue over the surface of the composite layer composed of elastomeric material and biomaterial (hydroxyapatite or bioglass); 4 - Biomaterial particles - bioglass or hydroxyapatite, dispersed in the volume of the continuous layer of adhesive material (elastomeric); 5 - Biomaterial particles - bioglass, dispersed in the volume of a ceramic material (alumina); 6 - Layer of newly formed tissue over the surface of the solid substrate ceramic composite with the biomaterial (bioglass) dispersed in its volume.
Figure 2 shows a schematic illustration of the discontinuous layer in the two possible situations: substrate with higher melting point than the biomaterial particles and substrate with lower melting point than the biomaterial particles. 1 - Original bioglass particles; 2 - Bioabsorbable polymer layer used as initial "adhesive"; 3 - Bioglass particles softened by thermal treatment and directly adhered to the solid substrate with high melting point (metal, ceramic or composite); 4 - Layer of newly formed tissue in interface with the solid substrate.
Figure 3 shows images obtained through scanning electron microscopy showing the range of precipitation of hydroxyapatite from the F18 bioglass particles after a bioactivity test.
Figure 4 graphically represents the distribution of the fraction of the area occupied by the particles on the surface (%) as an x and y function of the stroke (mm) after 5 coatings.
Figure 5 shows the fraction of coated area as a function of the number of coatings in the airbrushing coating method.
Figure 6 shows the water-titanium contact angle variation according to the surface condition of titanium.
Figure 7 shows particles adhered to the titanium surface after a micro-indentation adhesion test.
Figure 7a shows the presence of cracks caused by a 200gf vickers micro-indentation.
Figure 7b shows the same particle from (a), emphasizing that the cracks generated did not affect the adhesion of the particles even after 24 hours of bioactivity testing.
Figure 8 shows the osteoblastic cells adhered to the smooth titanium surfaces after three days of cultivation. The dark grey and light grey fluorescent sections indicate, respectively, an actin cytoskeleton, marked with phalloidin combined with Alexa Fluor, and cell nucleus, marked with DAPI ((4,6-diamidino-2-phenylindole).
Figure 8a shows titanium with no coating.
Figure 8b shows titanium coated with 45S5 bioglass.
Figure 8c shows titanium coated with F18 bioglass.
Figure 9 graphically represents the viability of osteoblastic cells over time, over the titanium surfaces, with and without coating.
Figure 10a shows the aspect of the smooth titanium implant before thermal treatment.
Figure 10b shows the aspect of the smooth titanium implant after thermal treatment with an average coating fraction of 8%.
Figure 10c shows the aspect of the rough titanium implant after thermal treatment with an average coating fraction of 8%.
Figure 11 shows the surface of a coated dental implant with an average coating fraction of 25%.
Figure 12 shows the top of the thread crest of an implant coated after the insertion and removal test.

### DETAILED DESCRIPTION OF THE INVENTION

The invention relates to a discontinuous coating composed of a biomaterial (which can be a bioceramic, biopolymer or bioactive composite), bioabsorbable and bioactive applied over a solid substrate. It refers, therefore, to a discontinuous layer which will be absorbed by the body during the tissue scarring process which ensures a lack of interfaces (potential cause of flaws), and direct contact of the solid substrate (implant) with the newly formed tissue as shown schematically on Figure 2.

### FIXATION OF PARTICLES TO THE SURFACE - LAYER FORMATION, DISCONTINUOUS

Material used: biomaterial in particle form, preferably bioglass.

Fixation method: temperature increase for situations in which the substrates have higher melting point than the biomaterial to be deposited and use of bioabsorbable polymeric gel as an adhesive substance for situations in which the substrates have lower melting point than the biomaterial to be deposited.

### COATING PARAMETERS

### POWDER QUANTITY

The definition of the amount of powder to be fixed to the surface is based on the answer to the following question: what is the minimum fraction of particles that produce, after a bioactivity test, a hydroxyapatite layer that coats 100% of the surface.

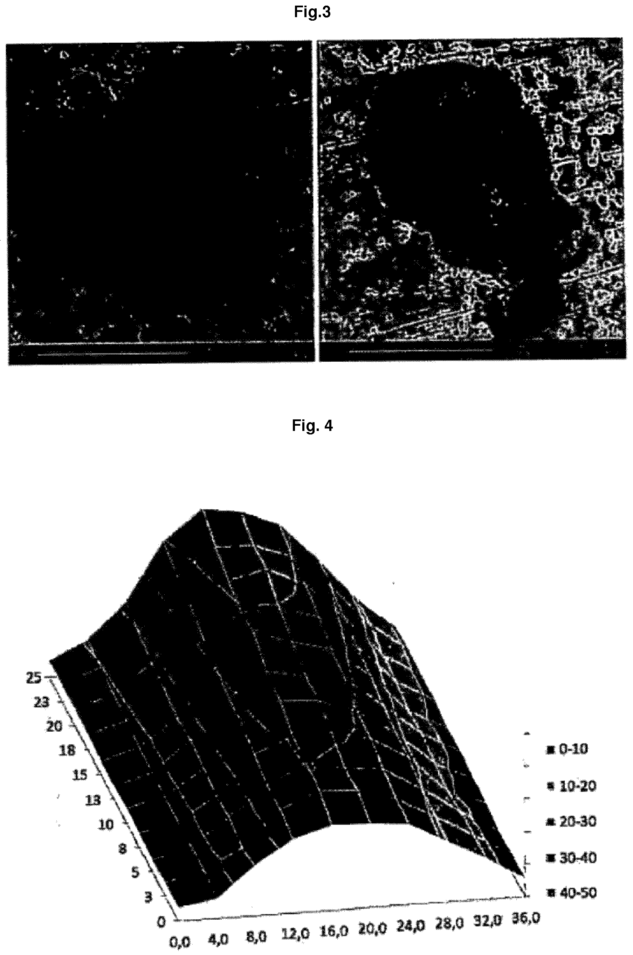

The results of these tests have shown that the range of precipitation of hydroxyapatite from the edge of the particles depends on the particle size distribution of the bioglass powder and its composition. For the bioglass used, the F18 bioglass, with a particle distribution between 10 and 100um, this distance was approximately equal to the particle diameter, as seen in Figure 3. This result indicated that if 25% of the surface is homogeneously covered by particles, it will be 100% covered by hydroxyapatite after the bioactivity test. The bioactivity tests were carried out based on the ISO 23.317 (2007) standard "Implants for surgery - *in vitro* evaluation for apatite-forming ability of implant materials".

Usage of other types of bioglass with various powder grain sizes changes the coated fraction, which leads to maximum formation of hydroxyapatite in the bioactivity test due to differences in particle speed dissolution. Thus, it was possible to determine that, if regions of bioactivity test with average diameter between 1 and 1200µm coat surface fractions between 2 and 80%, the aforementioned condition (surface 100% coated by hydroxyapatite after bioactivity test) will be met.

### COATING METHOD

Based on previously established criteria, five methods of transferring biomaterial to the solid substrate may be used:
1. Brush painting.
2. Drip coating
3. Dip Coating
4. Airbrushing or air atomizing.
5. Thermal spray.

### EXAMPLE OF COATING PROCESS OF TITANIUM DISCS THROUGH AIRBRUSHING

Titanium discs with 10mm diameter and 3mm thickness were used in this example. The transfer of biomaterial to the surface of the solid substrate, or coating, used biomaterial particles with particle size distribution between 5 and 500µm.

There is no information in the literature of the invention regarding the airbrushing method in the field of embodiment of this invention. For this reason, some parameters were necessary for this process in order to make it repeatable. Initially, the term "stroke" was established. In the graphic and art fields, in which the airbrush is extensively used, "stroke" refers to the pattern of the deposited paint - more or less dense, more or less wide, left by the airbrush in the work surface. In the current situation, the stroke shall be the particle deposition pattern over the titanium surface, characterized by the particle distribution and fraction delivered to the surface by the airbrush. This deposition pattern shall be determined by the following parameters:
1. Stroke density - average fraction of the surface area covered by the particles delivered to the surface during the first coating.
2. Final Stroke Density or work density - final average fraction of the surface area covered by the particles delivered to the surface. As many coatings as needed may be used to reach the final density.
3. Stroke Width - region in which the area fraction range covered by the particles delivered to the surface has the same values as the final stroke density.

The characteristics of the stroke used in this example are graphically presented in Figure 4. Its main characteristics were:
- Stroke Width: 1 to 40mm
- Stroke density: 1 to 5%
- Work density for 4 coatings: 25 to 30%
- Work density for 5 coatings: 45 to 50%
- Work density for 6 coatings: 50 to 60%

Through Figure 4, it is already possible to note that, after approximately 18 mm, the stroke already yielded the work density, which ranges from 40 to 50% for 5 coatings. From 25 mm, the stroke already has approximately 10 mm, which is the diameter of the titanium discs to be coated, and larger than the 3 mm average diameter of the real implants used in the subsequent application example in this work.

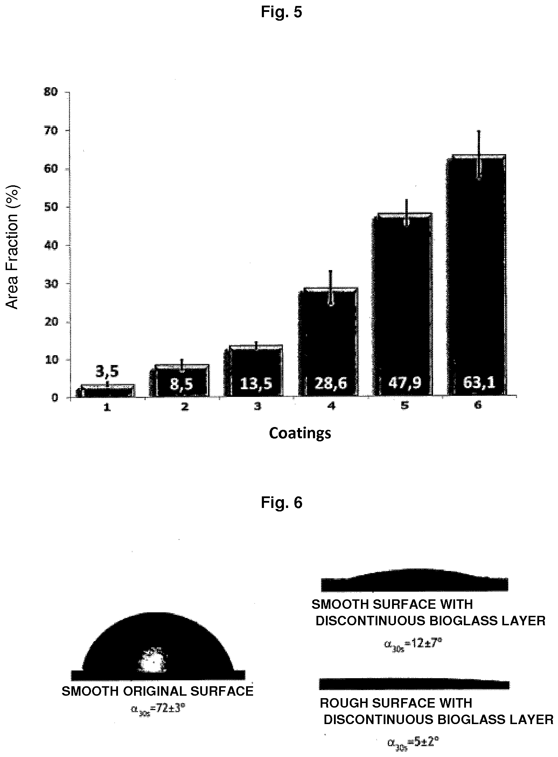

Figure 5 shows that the amount of particles delivered to the surface is not linear and varies from 3.5% of area fraction for one coating up to 60% for six coatings.

The other parameters used in airbrushing were:
- Air pressure between 0.5 and 3 pascal, preferably 1 pascal,
- Distance from the nozzle to the surface between 10 and 40 mm, preferably 20mm,
- Nozzle needle between 60 and 120 µm,
- Applying speed between 5 and 100 mm/s,
- For situations of dental implant coating, said implants must be positioned at a 0 to 45° horizontal angle and under rotation from 0.5 to 4 rpm,
- Relative air humidity between 44 and 49%,
- Temperature between 19 and 28°C.

The study of this example shows that it is possible to adjust a continuous layer production technique, such as airbrushing, in order to generate a discontinuous layer. Furthermore, it also shows that it is possible to project discontinuous layers with any biomaterial fraction. In this example, if generating discontinuous layers with 25% of the surface coated with bioglass is needed, 4 coatings should be applied.

### CHARACTERIZATION OF TITANIUM DISC COATINGS CONTACT ANGLE (WETTING)

The ability of bone-integrated dental implants to withstand loads depends on the quality and quantity of bone tissue connected to the implant that, in turn, is strongly influenced by the implant surface characteristics. Hydrophilic surfaces improve bone apposition and provide a quicker bone-implant contact.

Countless *in vitro, in vivo* and clinical results have shown the importance of this wetting or contact angle test for the dental implant field.

Table 1 shows the results of contact angle measurements for various conditions of the titanium surface.

From the samples coated with the F18 bioglass, the rough surface obtained through double acid etching featured the lowest contact angle. The angle value obtained was 5±2°. Figure 6 shows the variation of the contact angle due to variation of some parameters. It was possible to evidence that the coating of surfaces with bioglasses significantly decreased the contact angle of the titanium surfaces.

Most implant surfaces available at the market have contact angles higher than 10°. A few examples, including one national version, have contact angles around 5°. Nevertheless, it is possible to find in the market an implant with zero contact angle, in other words, with full wetting and that leads to better bone integration results than surfaces with higher wetting angles. These implants are manufactured by a worldwide leading company in the manufacture of dental implants.

**table 1 - Results of contact angle assessments between distilled water and the various conditions of the titanium surface**

| **Sample** | **Contact Angle (º)** | **Bioglass Fraction (%)** |
|---|---|---|
| Smooth, coated with F18 bioglass | 8.9±1 | 9.9±1 |
| Smooth, coated with F18 bioglass | 11.0±4 | 25.0±1 |
| Smooth, not coated | 54.5±8 | -- |
| Rough, not coated | 25.0±1 | -- |
| Rough, coated with F18 bioglass | 5.3±2 | 7±1 |
| Smooth, coated with 45S5 bioglass | 3.1±1 | 23±6 |
| Rough, coated with 45S5 bioglass | 0.8±0.6 | 17±5 |

### ADHESION EVALUATION OF BIOGLASS ISLANDS TO THE SUBSTRATE

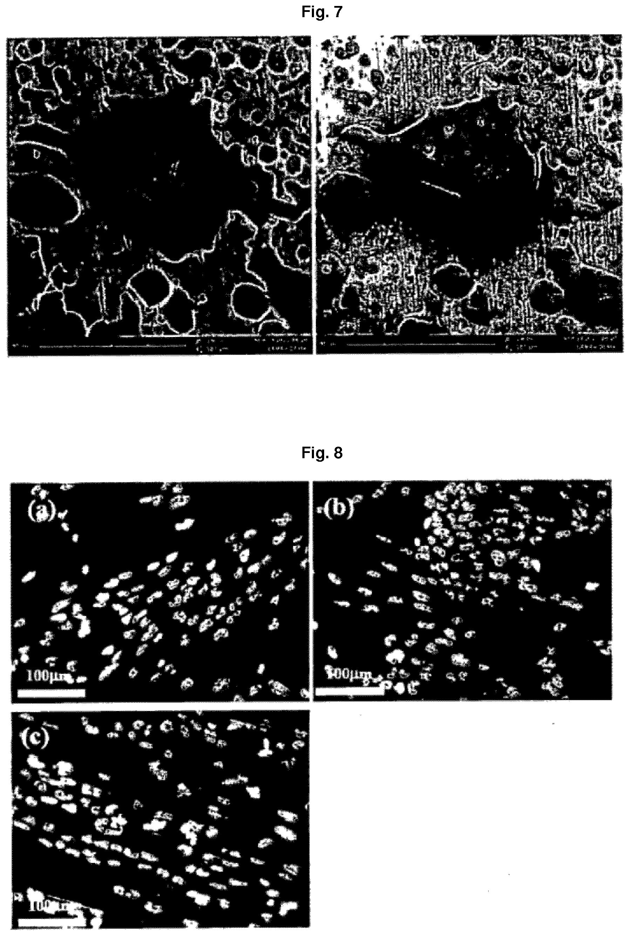

Micro-indentations with 200gf loads were generated on the surfaces of the bioglass islands fixed to the solid substrates. The objective was to quantitatively evaluate interface adhesion, an important parameter for placement dental implants. On Figure 7, no cracks were noticed on the titanium-bioglass interface, which enabled partial or full particle detachment. All cracks observed were perpendicular to the surface. This behavior is an indication of good adherence between titanium and bioglass.

### IN NITRO TESTS: CELL ADHESION AND CYTOTOXICITY

The objective of *in vitro* tests is to evaluate the biocompatibility of the biomaterial in a biological environment. Biocompatibility is related to the acceptance of a biomaterial by the nearby tissue and by the body as a whole, in other words, it determines if the performance of the biomaterial is compatible with the expectations and does not harm the patient/user.

### SAMPLES

Smooth titanium discs were coated by airbrushing as described in the section "Example of coating process of titanium discs through airbrushing" to be tested *in vitro.* Smooth titanium discs with no coating were tested for control purposes.

### TESTING CONDITIONS

For *in vitro* tests, a strain of tumor cells established from human osteosarcoma, named SaOS-2 was used (origin: Banco de Células do Rio de Janeiro - BCBRJ).

The cytotoxicity evaluation was carried out through the MTT method, or metabolic oxidization of 3-(4,5-dimethylthiazol-2-yl)-2,5 diphenyltetrazolium bromide (MTT of Sigma company - Saint Louis - MO - EUA) for 1, 3 and 5 days. The experiment was performed in triplicate.

The cell adhesion test evaluated the viability of cells adhered to the surface after 4 hours of incubation. After this time, samples were taken from the incubator and the cells were enzymatically detached from the titanium surfaces.

### CELL ADHESION

Figure 8 shows images obtained through confocal laser scanning microscopy. It includes cells adhered to the titanium surfaces after three days of cultivation. Note that the cells exhibit cytoplasmic extensions, which seek anchoring in various directions, mainly in the direction of surface lines. In (c), cell alignment with parallel features of superficial ridges is more clearly noted, and also highlights the set of various cells adhered to the surface emphasizing the joining of various cytoplasmic extensions and, consequently, of various cells. This hints at a good cell spread and adhesion, and evidences surface biocompatibility. It should be noted that there is virtually no morphological differences, or cell amount or distribution between the three surfaces.

### CYTOTOXICITY

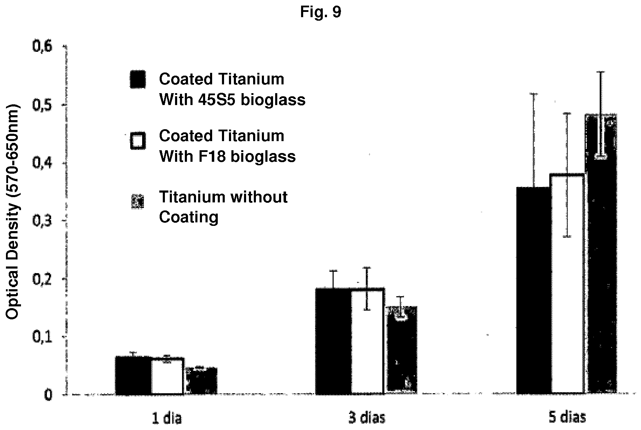

According to the results produced by the MTT test (Figure 9), it was observed that the coated surfaces sustained the viability of the osteoblastic cells, SaOS-2, throughout the cultivation time.

No significant statistical differences were observed between the tested samples - coated or not coated. For this reason, the coated surfaces analyzed have no cytotoxic effect, since according to the ISO 10993-5 standard "Biological evaluation of medical devices Part 5: Tests for *in vitro* cytotoxicity", the cytotoxic effect is verified where there is reduction of at least 30% in cell viability in relation to the known pattern - which is pure titanium in this case.

### EXAMPLE OF DENTAL IMPLANT COATING BY AIRBRUSHING

The option for testing airbrushing coating on dental implants was due to its higher geometric complexity and reduced sizes in comparison to orthopedic implants, for example.

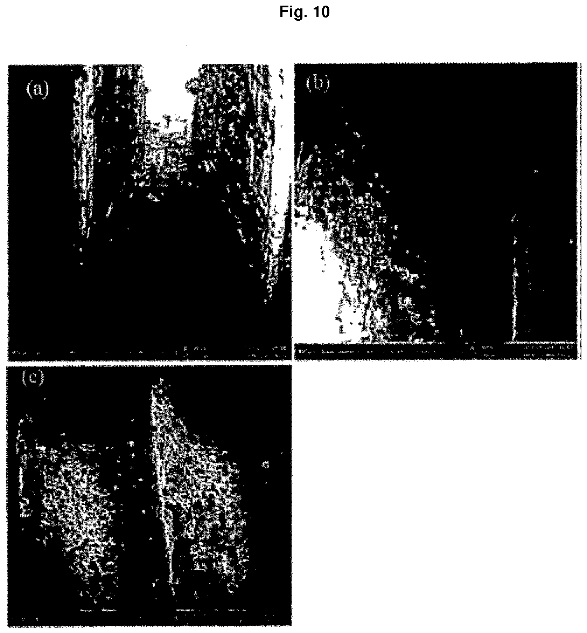

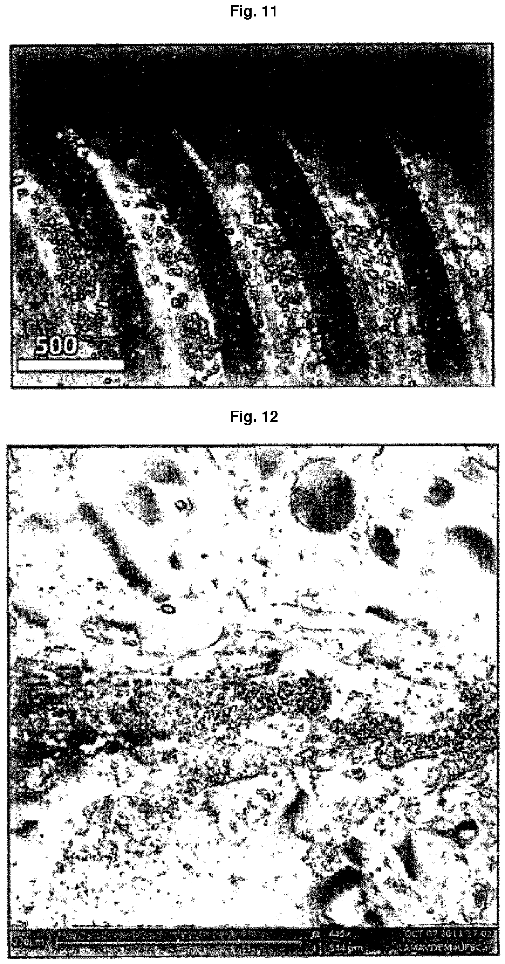

Figure 10 shows two surfaces covered with F18 bioglass with an average coating fraction of 8% and Figure 11 shows the surface of an implant with an average coating fraction of 25%.

The implant coatings used the same parameters used for coating the aforementioned titanium discs.

### CHARACTERIZATION OF IMPLANTS COATED WITH AIRBRUSHING

### SURFACE OF THE IMPLANTS

It should be highlighted that, aside from smooth surfaced implants, implants with double acid-etched surfaces - or rough - were coated. This surface is the best surface currently available commercially. Rough surfaces coated with bioglass, as already presented, have excellent wetting (low contact angle), which is very desirable for a bone integrating implant.

### HOMOGENEITY AND AREA FRACTION

The homogeneity analysis was based in approximately 200 images. Coated dental implants featured an average coating area of 25%.

### ADHESION EVALUATION OF BIOGLASS ISLANDS TO THE DENTAL IMPLANT

As the dental implant is a "bone screw", the ASTM F-543:2007 standard - Attachment A2 ("Test Method for Driving Torque of Medical Bone Screws") was used as test reference. Based on this standard, the implant insertion was simulated, but under more severe test conditions. These conditions were: (1) test performed in both ways, in other words, insertion and removal of the implant; (2) the process was carried out on a dry basis and (3) on a material that emulates the cortical bone - denser, and generally present in just part of the insertion depth of the implant. Thus, it is expected that, if the coating withstands these test conditions, it will certainly withstand the real insertion process.

The parameters of this test, based on the ASTM F-543:2007 standard - Attachment A2, basically relate to the standardization of the test block. Thus, the testing parameters were:
- Test block material: 40 degree polyurethane with equivalent density and properties to a cortical bone.
- Block dimensions: 100mm height, 40mm diameter. The standard indicates that the lowest dimension must be higher than 10x the nominal diameter of the screw to be tested, and minimum 4.8mm height.
- Screw insertion hole: conditions as provided by the manufacturer - for this test, 2mm up to a 9mm depth and 2.8mm up to a 7mm depth, for a 3.5mm diameter implant.

As evidenced on Figure 12, no detachments or cracks of the coating features were found. In addition, based on these results, it can be stated that the coating adhesion over the implants shall not be affected by the actual use conditions, since the conditions of this test are much more severe than the real conditions.

### CONCLUSIONS

### ABOUT THE COATING EXAMPLE ON TITANIUM DISCS

The airbrushing coating process is viable for obtaining discontinuous coatings with any desired final fractions, as it was possible to project and produce coatings in an accurate and reproducible manner.

By this example of suitability of a method for obtaining a continuous layer - airbrushing - it may be stated that it is possible to obtain a discontinuous layer through any continuous layer deposition method, upon correct adjustment of the respective process parameters.

The addition of the F18 bioglass layer has generated a surface with a contact angle on par with the best implants currently on the world market, in other words, θ<5°.

The results obtained on *in vitro* tests show that the discontinuous layer obtained may provide significant improvements to the general performance of implants (specially orthopedic and dental implants).

### ABOUT THE COATING EXAMPLE ON TITANIUM DENTAL IMPLANTS

The bioglass layer applied over the titanium dental implant surface was found to be very homogenous. The adhesion of particles to the metallic substrate was suitable, as the dental implant, although tested under more severe conditions than the real use conditions - from the viewpoint of shearing forces, showed no flaws on its layer.

## Claims

1. Process of discontinuous coating using a bioabsorbable and bioactive material on solid substrates, wherein it comprises the steps of:
- Selection of biomaterial;
- Transfer of biomaterial to the surface of the implant;
- Fixation of biomaterial particles to the surface of the implant.

2. Process of discontinuous coating, according to claim 1, wherein the biomaterial is a bioceramic, a biopolymer, a composite or a bioglass.

3. Process of discontinuous coating, according to claims 1 or 2, wherein the biomaterial is, preferably, a bioglass.

4. Process of discontinuous coating, according to any one of claims 1 to 3, wherein the biomaterial is applied to the surface of the solid substrate in an island format with size distribution of 1 and 1200 µm.

5. Process of discontinuous coating, according to any one of claims 1 to 4, wherein the amount of biomaterial powder to be used fills between 2 and 80% of the surface of the solid substrate.

6. Process of discontinuous coating, according to claim 1, wherein the transfer step of biomaterial particles to the surface is done by thermal spray, airbrushing or air atomizing, drip coating, dip coating or brush coating.

7. Process of discontinuous coating, according to claim 6, wherein the transfer step is preferably done by airbrushing or air atomizing.

8. Process of discontinuous coating, according to claim 7, wherein the parameters used for airbrushing are:
- Stroke Width: 1 to 40mm,
- Stroke density: 1 to 5%,
- Work density for 4 coatings: 25 to 30%,
- Work density for 5 coatings: 45 to 50%,
- Work density for 6 coatings: 50 to 60%,
- Air pressure between 0.5 and 3 pascal, preferably 1 pascal,
- Distance from the nozzle to the surface between 10 and 40 mm, preferably 20mm,
- Nozzle needle between 60 and 120µm,
- Applying speed between 5 and 100 mm/s,
- In case of symmetric implants or substrates, the coating may be done in rotation from 0.5 to 4 rpm, preferably 1 rpm, and at an angle between 0 and 45° horizontally,
- Relative humidity between 44 and 49%,
- Temperature between 19 and 28°C.

9. Process of discontinuous coating, according to claim 1, wherein at the fixation step when the solid substrate has a higher melting point than the biomaterial being applied, the biomaterial is fixed directly to the substrate.

10. Process of discontinuous coating, according to claim 9, wherein the biomaterial is fixed to the solid substrate through temperature elevation to a level that is higher than its vitreous transition temperature.

11. Process of discontinuous coating, according to claim 1, wherein in the fixation step, when the substrate features a lower melting point than that of the biomaterial, the biomaterial may be fixed to the surface through a polymer-based bioabsorbant adhesive gel layer.

12. Process of discontinuous coating, according to any one of claims 9 to 11, wherein the bioabsorbable gel is, preferably, HPMC (hydroxypropyl methylcellulose) or collagen in the concentration of 0.05 to 1.2g for each liter of the mixture containing 30 to 50% of water and 50 to 70% of ethyl alcohol.

13. Process of discontinuous coating, according to any one of claims 9 to 12, wherein the biomaterial is dispersed in the polymeric gel in the concentration of 0.001 to 0.6g/ml.

14. Discontinuous coating of a bioabsorbable and bioactive material on solid substrates, wherein it is obtained through the process defined on claims 1 to 13.

15. Discontinuous coating, according to claim 14, wherein said coating is totally consumed in up to 10 days after implantation.

16. Discontinuous coating, according to claims 14 or 15, wherein it promotes interface between the newly formed tissue and said solid substrate and local stimulation for growth of said tissue.

17. Use of a discontinuous coating, wherein it applies to dental, hip and knee implants, bone reconstruction plates, intervertebral spacers, pedicular screws of spinal implant systems, repair of anophthalmic sockets, and any implantable solid substrate.

18. Use of a discontinuous coating, according to claim 17, wherein it applies, preferably, to orthopedic and dental implants.
